Europäisches Patentamt

European Patent Office

Office européen des brevets

(19)

(11) Publication number: **0 257 824**
**A2**

(12) **EUROPEAN PATENT APPLICATION**

(21) Application number: 87306735.9

(51) Int. Cl.⁴: **A47K 10/16**

(22) Date of filing: 30.07.87

(30) Priority: 06.08.86 JP 186100/86
06.08.86 JP 186101/86

(43) Date of publication of application:
**02.03.88 Bulletin 88/09**

(84) Designated Contracting States:
**CH DE FR GB IT LI**

(71) Applicant: **KOBAYASHI PHARMACEUTICAL
CO. LTD.
25, Doshomachi 5-chome
Higashi-ku Osaka(JP)**

(72) Inventor: **Kashima, Kenji
7-28, Tokuraishi 1-chome
Toyonaka-shi Osaka(JP)**
Inventor: **Iwamoto, Shyuei
9-60, Nigawakita 2-chome
Takarazuka-shi Hyogo(JP)**

(74) Representative: **Barker, Rosemary Anne et al
Barlow, Gillett & Percival 94 Market Street
Manchester M1 1PJ(GB)**

(54) **Paper and liquid sprayer for wiping clean toilet seat.**

(57) A water decomposable paper (I) impregnated with an organic solvent is used to wipe clean a toilet seat and at the same time is effective to disinfect and eliminate germs from the seat surface. Water or another organic or inorganic solvent may be added to the organic solvent. Also, a bactericide may be added for disinfecting purposes. The paper may be flushed down the toilet after it has been used for wiping.

A sprayer comprising a spray vessel (I2) containing organic solvent (I3) may be used to spray the latter directly onto the toilet seat prior to wiping or onto the wiping paper as required.

EP 0 257 824 A2

## PAPER AND LIQUID SPRAYER FOR WIPING CLEAN TOILET SEAT

This invention relates to a clean-wiping paper for disinfecting a toilet seat surface and capable of being flushed down the toilet, and a sprayer for spraying liquid directly over such a surface or onto a tissue paper in connection with clean-wiping the surface for disinfecting purposes.

Recently, a tissue paper called "prewetted tissue" has become widely used. Generally this is a piece of tissue paper or nonwoven cloth which is pre-impregnated with water or other liquid and packaged as such. Since it is pre-moistened, such a tissue paper is handy and is often used for wiping or cleaning the hands or face or for other personal purposes.

The sanitary condition of a toilet seat is a matter for concern to the user because a part of his skin is to go into direct contact with the seat. Therefore, toilet users often wipe the surface of the toilet seat with aforesaid "prewetted tissue" prior to sitting on the seat. However, such a "prewetted tissue" can only remove superficial dirt and, according to the findings of our survey, it has little or no bactericidal or germ-eliminating effect, it being impossible to wipe the toilet seat surface substantially clean with such a tissue. The term "germ-eliminating" referred to herein means eliminating germs from a toilet seat serface by wiping the surface.

Such a "prewetted tissue" is often of a nonwoven cloth or of a paper of such type that can hardly be dispersed in water, and as such, if it is flushed down the toilet after it is used for wiping the toilet seat surface, it may cause pipe clogging or adversely affect the sewage purifier. Naturally, therefore, such tissue is not suited for being flushed down the toilet.

When no "prewetted tissue" is in hand, it is a usual practice to wipe the toilet seat with an ordinary tissue paper, if personally carried, or with a cut from a roll of toilet paper provided in the toilet room; but with any such paper, which has no germicidal or germ-eliminating performance, it is apparent that the toilet seat cannot be wiped to the satisfaction of any reasonable sanitary requirements.

This invention is intended to solve aforesaid problems. Accordingly, it is an object of the invention to provide a clean-wiping paper which can wipe dirts off the surface of a toilet seat, and which has excellent germicidal and germ-eliminating performance.

It is another object of the invention to provide a clean-wiping paper which can be flushed down the toilet after it is used for seat surface cleaning.

It is a further object of the invention to provide a spray unit which sprays a certain liquid over the surface of a toilet seat directly or onto such soft material as toilet paper, tissue paper, cloth, or the like so as to be able to disinfect the seat surface or wipe it clean of germs.

With the above objects in view, a first aspect of the invention comprises a water decomposable paper impregnated with an organic solvent.

The term "water decomposable paper" referred to herein means a paper whose component fibers have at least such interfiber bond strength as is only necessary for paper formation and maintenance of a wiping function in their dry condition, but which, in wet condition or when for example immersed in water, is extremely lowered in interfiber bond strength and easily decomposed or dispersed in water when subjected to any external force. In other words, it is characteristically similar to such kind of paper as is used as ordinary toilet paper, for example.

A water decomposable paper useful for the purpose of the invention comprises rayon and/or pulp, and in mixture therewith, polyvinyl alcohol (PVA) and/or carboxymethyl cellulose (CMC). It is understood, however, that the type of the water decomposable paper useful for the purpose of the invention is not limited to the aforesaid one, and that it embraces all sorts of paper having above mentioned characteristics, that is, water decomposability. In said water decomposable paper, the blend ratio of components is suitably determined so that the paper has a strength enough to permit hand wiping operation when in such condition as it is impregnated with an organic solvent which will be hereinafter described, whereas the paper, in wet condition or when for example immersed in water, is easily decomposed or dispersed in water if subjected to any external force.

Preferred organic solvents useful in the invention include alcohols, such as methanol, ethanol, n-propanol, and isopropanol; and hydrocarbon halides, such as I, I, 2-trichloro-I, 2, 2-trifluoroethane and the like. These organic solvents should be of such type as will have no or little effect on human health when it goes in contact with human skin.

Above mentioned organic solvents have bactericidal and/or germ-eliminating activity; therefore, by virtue of their action, it is possible to remove not only dirts, but various germs as well. They may be used either alone or in combination.

As one advantageous development of the invention, it is possible to impregnate the water decomposable paper with a mixture of any such organic solvent with water. In this way, timing can be easily adjusted for evaporation of the organic solvent deposited on the toilet seat until drying thereof. Another advantage of this arrangement is that since the water decomposable paper contains water, it has increased softness and also improved touch. Another advantage is that the use of the organic solvent can be economized, which leads to cost reduction. Addition of water in a suitable quantity has no negative effect on the bactericidal or germ-eliminating activity of the organic solvent.

As another advantageous development of the invention, it is possible to add some other kind of solvent to aforesaid organic solvent. Such addition produces a further favorable effect. Preferred other kinds of solvents useful for this purpose are polyhydric alcohols, such as glycerin and propylene glycol; esters, such as isopropyl palmitate and the like; and inorganic solvents, such as silicone oil and the like. Addition of glycerin or propylene glycol produces the effect of preventing moisture volatilization from the hand or fingers when the paper is pinched while improving moisture retention, that is, emollient effect. It is also effective for preventing skin degreasing due to the organic solvent. The addition of isopropyl palmitate provides the advantage that the surface of the toilet seat after being wiped is prevented from becoming sticky. The addition of silicone oil provides, ·in addition to such advantage, the advantage that the bactericidal effect of the solvent mixture can be retained longer.

As a further advantageous development of the invention, it is possible to have greater assurance of such bactericidal effect by adding a bactericide to aforesaid organic solvent. Such addition is desirable in that a very sanitary condition can be created. Bactericides useful for the purpose of this invention are not particularly limited insofar as they are soluble in the above mentioned organic solvents. Various known bactericides may be used, including, for example, cationic surface active agents, such as benzalconium chloride, benzetonium chloride, and cetylpyridinium chloride, anionic surface active agents, such as sodium N-lauroyl sarcosinate and the like, amphoteric surface active agents, such as alkyldiaminoethylglycin hydrochloride and the like, organic acids, such as sorbic acid and the like, parabenes, such as ethyl parahydroxybenzoate and the like, phenols, such as cresol, o-phenylphenol, p-chlorophenol, parachloroxylel, and 2, 4, 4'-trichlor-2'-hydroxydiphenyl ether, and sulfur-containing substances, such as chlroamine T and 2-(4-thiazoyl)benzimide. Also, trichlorocarbanilide and hexachlorophene may be used.

According to a second aspect of the invention, a liquid sprayer comprising any of aforesaid organic solvents, or a liquid consisting of a mixture of such organic solvents and other kinds of solvent or a bactericide, contained in a spray vessel is provided. For the spray vessel may be used any known one of the type which is used with liquefied petroleum gas, carbon dioxide gas, or the like spray material. A hand-pump type sprayer may also be used.

The sprayer is preferably of a compact portable size so that it may be conveniently personally carried during an outing; thus, the disinfecting liquid may be sprayed onto any tissue paper or toilet paper, or sprayed directly onto the surface of a toilet seat, in connection with wiping the seat surface as required, whereby the seat surface can be readily wiped clean.

The invention will be described further by reference to the accompanying drawings, in which:

FIG. I is a perspective view showing a clean-wiping paper according to the invention;

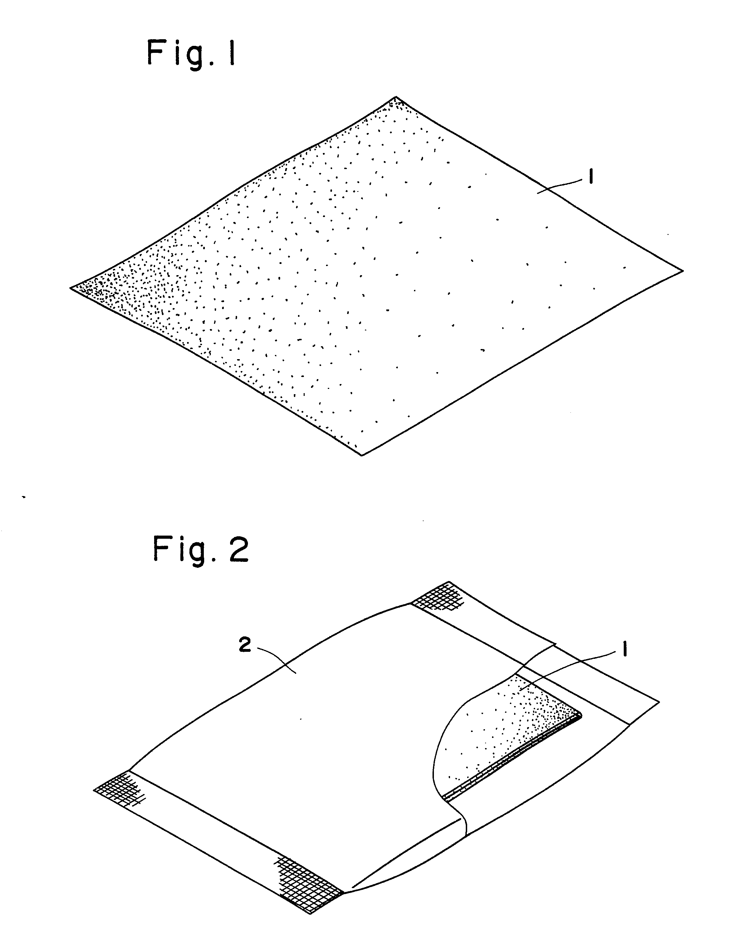

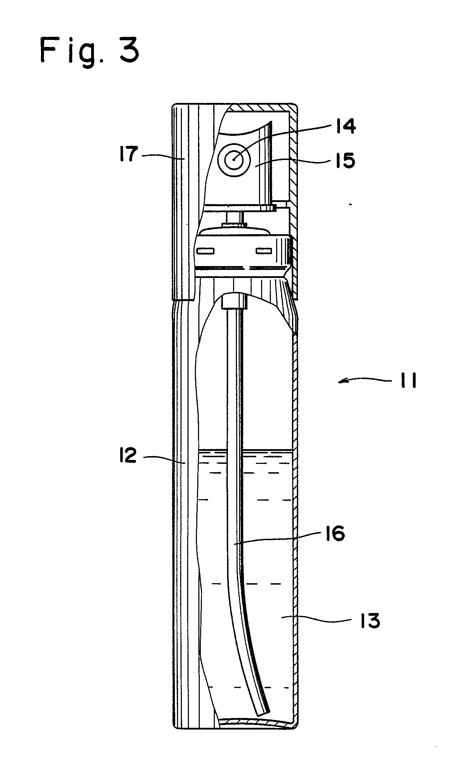

FIG. 2 is a partially cutaway view in perspective showing the clean-wiping paper as it appears when finely folded and sealdly packed; and

FIG. 3 is a partially cutaway perspective view showing one form of sprayer embodying the invention.

In FIGs. I and 2, numeral I designates the toilet seat wiping paper in accordance with the invention, which is finely folded and sealedly packed in a polyethylene or aluminum foil package 2 for ease of carrying. The clean-wiping paper I comprises a water decomposable paper impregnated with an organic solvent or a liquid including same. If necessary, suitable additives, such as a perfume and a deodorizer, may be added to the organic solvent.

FIG. 3 shows the sprayer in accordance with the invention. The sprayer II comprises a spray vessel I2 and a liquid I3 contained therein which consists of or includes the same organic solvent as is impregnated into the aforesaid clean-wiping paper I. The quantity of liquid contained in the spray vessel I2 varies according to the kind of the liquid I3, but preferably it is 30 - 70 parts by volume relative to the volume of the spray vessel I2. The spray vessel I2 is of the type which is conventionally used with liquefied petroleum gas or carbon dioxide gas; but needless to say, it may be of the hand pump spray type.

A manipulating member I5 having a nozzle I4 is provided at the top end of the spray vessel I2. A finger push on the manipulating member I5 allows the interior liquid I3 to be sprayed from the nozzle I4 through an introduction pipe I6. When the sprayer is not in use, the manipulating member I5 is covered with a cap I7.

The following examples are given to further illustrate the invention.

In each of the examples given, the water decomposable paper is one of the following six varieties, A - F.

Water decomposable paper A : a mixture of rayon and PVA in a weight ratio of 95 : 5.

Water decomposable paper B : a mixture of rayon and CMC in a weight ratio of 60 : 40

Water decomposable paper C : a mixture of pulp and PVA in a weight ratio of 90 : 10.

Water decomposable paper D : a mixture of pulp and CMC in a weight ratio of 60 : 40.

Water decomposable paper E : a mixture of rayon, pulp, and PVA in a weight ratio of 67 : 28 : 5.

Water decomposable paper F : a mixture of rayon, pulp, and CMC in a weight ratio of 30 : 20 : 50.

Example I

A clean-wiping paper is prepared by using an ethanol denatured by saccharose octacetate as an organic solvent, said organic solvent being impregnated into aforesaid water decomposable paper A. The bactericidal or germ-eliminating effect of this clean-wiping paper was examined in the following manner:

Viable count of general germs on the surface of a toilet seat in each of various public facilities was first examined by using a standard agar-agar impression medium for viable count determination. Then, the toilet seat was wiped clean with the clean-wiping paper; thereafter, viable count of germs was examined by using said impression medium. Examination results are shown in Table I. It is noted that the decrease in count (%) for each case was determined on the basis of the following equation after mean values of pre-wiping and post-wiping viable counts shown in Table I were calculated.

$$\text{Decrease in count (\%)} = \left(1 - \frac{\text{post-wiping count}}{\text{pre-wiping count}}\right) \times 100$$

Table I

| Example No. Site | 1 | |
|---|---|---|
| | Pre-wiping | Post-wiping |
| Dept store A | 900 - 1500 | 0 |
| Dept store B | 0 - 500 | 0 |
| Hotel H | 22000 - 41000 | 0 |
| Hotel T | 10000 - 51000 | 0 - 800 |
| Building M | 700 - 2100 | 0 |
| Hospital S | 1500 - 3500 | 0 |
| Decrease in count (%) | 99 | |

Examples 2 - 7

Clean-wiping papers were individually prepared by using organic solvents and water-decomposable papers in such combinations as indicated in Table TT. Decrease in count for each individual case was determined on the basis of measurements carried out as in Example I. In Table II, values for organic solvents are expressed in volume against their proportions used.

## Table II

| Example No. | | 2 | 3 | 4 | 5 | 6 | 7 |
|---|---|---|---|---|---|---|---|
| Organic solvent | Ethanol | | | | | 75 | 75 |
| | Methanol | 100 | | | | | |
| | n-propanol | | 100 | | | | |
| | Isopropanol | | | 100 | | 25 | . |
| | 1, 1, 2-trichloro-1, 2, 2-trifluoethane | | | | 100 | | 25 |
| Water-decomposable paper | | B | B | B | B | D | D |
| Decrease in count (%) | | 94 | 95 | 98 | 90 | 99 | 93 |

Examples 8 - 10

In each example, other solvent was added to an organic solvent or solvents and the mixture was impregnated into a water decomposable paper, a clean-wiping paper being thus prepared. Decrease in count in each case was determined on the basis of measurements made in same manner as in Example I. Values for such decrease are shown in Table III.

Table III

| Example No. | | 8 | 9 | 10 |
|---|---|---|---|---|
| Organic solvent | Ethanol | 70 | 70 | |
| | n-propanol | | | 20 |
| | Isopropanol | | 10 | 50 |
| | 1, 1, 2-trichloro-1, 2, 2-trifluoroethane | | | 10 |
| Other solvent | Water | 30 | 20 | |
| | Silicone oil | | | 10 |
| Water-decomposable paper | | D | D | A |
| Decrease in count (%) | | 100 | 99 | 96 |

As above seen, throughout Examples I - I0, the decrease in count was not less than 90%, it being thus apparent that the clean-wiping paper according to the invention has excellent disinfecting performance.

Examples II - 25

In each example, a bactericide was added to an organic solvent or solvents, and the mixture was impregnated into a water-decomposable paper, a clean-wiping paper being thus prepared.

Organic solvent/bactericide combinations are shown in Table IV. Values for decrease in count determined on the basis of measurements made in same manner as in Example I are also as shown in the table. In the table, values for the quantities of the individual bactericides used are in terms of grams, which means that the volume of each organic solvent to which such quantity of bactericide was added was I00 ml as a whole.

Table IV

| | Example No. | 11 | 12 | 13 | 14 | 15 | 16 | 17 | 18 |
|---|---|---|---|---|---|---|---|---|---|
| Organic solvent | Ethanol | 100 | 100 | 100 | 100 | 100 | 100 | 100 | 100 |
| | Isopropanol | | | | | | | | |
| Bactericide (g) | Benzalconium chloride | 0.01 | | | | | | | |
| | Benzetonium chloride | | 0.01 | | | | | | |
| | Cetyl-pyridinium chloride | | | 0.02 | | | | | |
| | Sodium N-lauroyl sarcosinate | | | | 0.05 | | | | |
| | Sorbic acid | | | | | 0.05 | | | |
| | Ethyl parahydro-xybenzoate | | | | | | 0.01 | | |
| | Cresol | | | | | | | 0.03 | |
| | p-chloro-phenol | | | | | | | | 0.02 |
| Water-decomposable paper | | A | A | E | F | F | F | E | E |
| Decrease in count (%) | | 95 | 99 | 97 | 95 | 93 | 96 | 95 | 98 |

Table IV (continued)

| Example No. | | 19 | 20 | 21 | 22 | 23 | 24 | 25 |
|---|---|---|---|---|---|---|---|---|
| Organic solvent | Ethanol | 60 | 60 | 100 | 100 | 100 | 100 | 100 |
| | Isopropanol | 40 | 40 | | | | | |
| Bactericide (g) | Trichloro-carbanilide | 0.01 | | | | | | |
| | Hexachlorophene | | 0.01 | | | | | |
| | O-phenylphenol | | | 0.01 | | | | |
| | Chloroxylenol | | | | 0.05 | | | |
| | 2, 4, 4'-trichloro-2'-hydroxydiphenyl ether | | | | | 0.03 | | |
| | 2-(4-thiazoly) benzimide | | | | | | 0.01 | |
| | Alkyldiamino-ethylglycin hydrochloride | | | | | | | 0.05 |
| Water-decomposable paper | | C | C | E | E | E | E | E |
| Decrease in count (%) | | 93 | 93 | 95 | 99 | 98 | 97 | 96 |

Examples 26 - 32

In each example, other solvents and a bactericide were added to an organic solvent or solvents and the mixture was impregnated into a water decomposable paper, a clean-wiping paper being thus prepared. Organic solvents/bactericides/water-dissolvable paper combinations and values for decrease in count are shown in Table V.

Table V

| | Example No. | 26 | 27 | 28 | 29 | 30 | 31 | 32 |
|---|---|---|---|---|---|---|---|---|
| Organic solvent | Methanol | | | | 10 | | | 5 |
| | Ethanol | 70 | 50 | 60 | 60 | 70 | 70 | 60 |
| | Isopropanol | | 40 | 35 | | | | 30 |
| Other solvent | Water | 30 | 10 | 5 | 20 | 25 | 25 | |
| | Glycerin | | | | | | 5 | |
| | Propylene glycol | | | | | 5 | | |
| | Isopropyl palmitate | | | | | | | 5 |
| Bactericide (g) | Benzalconium chloride | 0.05 | | | 0.01 | | 0.01 | 0.01 | |
| | Cresol | | | | 0.03 | | | |
| | Chloramine T | | 0.01 | | | | | |
| | O-phenylphenol | | | | | | | 0.01 |
| Water-decomposable paper | | D | A | E | E | D | D | A |
| Decrease in count (%) | | 100 | 98 | 99 | 99 | 99 | 99 | 100 |

In Examples 11 - 32 wherein a bactericide was added to the organic solvent or solvents, some improvement was observed in percentagewise decrease in count as compared with the earlier examples in which no bactericide was added, the rate of such decrease not being less than 93%.

Throughout Examples 1 - 32, there was no case in which the water-decomposable paper was broken during the process of impregnation with organic solvents, etc., or during toilet seat wiping operation. The paper was readily decomposed or dispersed when immersed and washed in water, there being seen no such trouble as pipe clogging.

The liquid used in Example I was contained in a sprayer embodying the second aspect of the invention. The liquid was sprayed directly onto the surface of a toilet seat and the surface was wiped with a toilet paper provided in the toilet room. The viable count in this case was same as in Example I, and it was also found that same degree of disinfecting effect as in Example I was obtainable. Similar effect was also seen when the seat surface was wiped with a toilet paper onto which the liquid was sprayed.

The sprayer in accordance with the invention exhibits excellent bactericidal or germ-eliminating performance, and therefore, with the sprayer it is possible to wipe the surface of a toilet seat to an almost perfect sanitary condition. Furthermore, it is handy to carry and easy to use.

For the purpose of comparison, measurements were made by using a known "prewetted tissue" having such ingredients in such proportions as indicated in Table VI and in the same manner as in Example I. The results are shown in Table VII. According to the results, the value for decrease in count is on the minus side, showing that the post-wiping count was larger than the pre-wiping count. This may be explained by the fact that because of the presence of irregularities on the surface of the toilet seat, germs were allowed to float up from recessed spots on the surface when the surface was wetted, thus resulting in increased viable count.

From a comparison with this reference example, it can be clearly understood what excellent disinfecting performance the clean-wiping paper of the invention does have.

### Table VI

| Component | Volume (%) |
|---|---|
| Surface active agent | 0.1 - 1 |
| Parabenes | 0.2 - 0.4 |
| Ethanol | 5 - 8 |
| Water | Balance |

### Table VII

| | Pre-wiping | Post-wiping |
|---|---|---|
| Dept store A | 2500 - 4100 | 3000 - 5300 |
| Dept store B | 800 - 1200 | 2400 - 3000 |
| Hotel H | 2800 - 8500 | 2500 - 5000 |
| Hotel T | 1800 - 7600 | 4900 - 6800 |
| Building M | 600 - 5000 | 2100 - 3300 |
| Hospital S | 1900 - 2600 | 4200 - 7300 |
| Decrease in count (%) | -26 | |

## Claims

1. Toilet seat cleaning paper comprising water-decomposable paper (l) impreganted with an organic solvent.

2. Paper according to claim l wherein the organic solvent is an alcohol or a hydrocarbon halide.

3. Paper according to claim l or 2 and impregnated with a mixture of an organic solvent and water.

4. Paper according to claim l, 2 or 3 and further impregnated with glycerin and/or propylene glycol and/or isopropyl palmitate, and/or silicone oil.

5. Paper according to any preceding claim and further impreganted with a bactericide.

6. Paper according to claim 5, wherein the bactericide is a surface active agent, an organic acid, a parabene, a phenol, or a sulphur-containing substance.

7. Paper according to any preceding claim wherein the water-decomposable paper (l) comprises rayon and/or pulp and, in mixture therewith, polyvinyl alcohol and/or carboxymethyl cellulose.

8. A sprayer comprising a spray vessel (l2) having a spray nozzle (l4) and an organic solvent (l3) contained therein, said sprayer being designed either for spraying the organic solvent directly onto the surface of a toilet seat which is then wiped clean with a soft material, such as paper or cloth, or for spraying the organic solvent onto such soft material which is then used to wipe clean the surface of the toilet seat.

9. A sprayer according to claim 8, wherein the organic solvent is an alcohol or hydrocarbon halide.

10. A sprayer according to claim 8 or 9 wherein the organic solvent includes a bactericide.

11. A sprayer according to claim 10, wherein the bactericide is a surface active agent, an organic acid, a parabene, a phenol, or a sulphur-containing substance.

12. Use of a sprayer, comprising a spray vessel (12) having a spray nozzle (14) and containing an organic solvent (13), in conjunction with water-decomposable paper to apply the organic solvent (13) to a toilet seat and wipe it clean.

Fig. 1

Fig. 2

# Fig. 3